# EUROPEAN PATENT SPECIFICATION

(11) **EP 1 297 181 B1**
(45) Date of publication and mention of the grant of the patent: **14.07.2010**
(21) Application number: 01954272.9
(22) Date of filing: 29.06.2001
(51) Int. Cl.: C12Q 1/68

(54) **SAMPLE GENERATION FOR GENOTYPING BY MASS SPECTROMETRY**
PROBENAUFBEREITUNG ZUR GENOTYPISIERUNG MITTELS MASSENSPEKTROSKOPIE
GENERATION D'ECHANTILLONS POUR GENOTYPAGE PAR SPECTROMETRIE DE MASSE

(30) Priority: 30.06.2000 EP 00401886
(43) Date of publication of application: 02.04.2003
(73) Proprietor: Commissariat à l'Énergie Atomique et aux Énergies Alternatives, 75015 Paris (FR)
(72) Inventor: GUT, Ivo, Glynne, F-75012 Paris (FR); LECHNER, Doris, F-75012 Paris (FR); SAUER, Sascha, F-75012 Paris (FR)
(74) Representative: Callon de Lamarck, Jean-Robert
(86) International application number: PCT/IB2001/001439
(87) International publication number: WO 2002/000931

(56) References cited:
- EP-A- 0 962 537
- WO-A-96/27681
- DE-A- 19 824 280
- US-A- 5 885 775
- GRIFFIN TIMOTHY J ET AL: "Direct genetic analysis by matrix-assisted laser desorption/ionization mass spectrometry." PROCEEDINGS OF THE NATIONAL ACADEMY OF SCIENCES OF THE UNITED STATES, vol. 96, no. 11, 25 May 1999 (1999-05-25), pages 6301-6306, XP002154776 May 25, 1999 ISSN: 0027-8424 cited in the application
- SAUER S ET AL: "A novel procedure for efficient genotyping of single nucleotide polymorphisms" NUCLEIC ACIDS RESEARCH, OXFORD UNIVERSITY PRESS, SURREY, GB, vol. 28, no. 5, 1 March 2000 (2000-03-01), page E13 XP002158531 ISSN: 0305-1048
- BERLIN KURT ET AL: "Analysis of negatively 'charge tagged' DNA by matrix-assisted laser desorption/ionization time-of-flight mass spectrometry." RAPID COMMUNICATIONS IN MASS SPECTROMETRY, vol. 13, no. 17, 1999, pages 1739-1743, XP000971118 ISSN: 0951-4198

## Description

The invention is drawn to a universal method for preparing DNA samples for genotyping of single nucleotide polymorphisms (SNPs), deletions and insertions by mass spectrometry. The method of the invention allows to generate samples in a way that allows a wide representation of the genome of the individual to be tested, and to analyze these samples by mass spectrometry without the need of a purification step.

The most important of the genome projects, the complete sequence of the human genome, will be finished in the next few years. This project will reveal the complete sequence of the 3 billion bases and the relative positions of all estimated 100.000 genes in this genome. Having this sequence opens unlimited possibilities for the elucidation of gene function and interaction of different genes. It also allows the implementation of pharmacogenetics and pharmacogenomics. Pharmacogenetics and pharmacogenomics aim at a targeted use of medication dependent on the genotype of an individual and so the dramatic improvement of the efficiency of drugs. A necessary intermediate step to this is the determination of variability of different individuals on a genome basis. This is accomplished by the determination of different markers and then using these for genotyping.

Currently two kinds of markers are used for genotyping: microsatellites and single nucleotide polymorphisms (SNPs). Microsatellites are highly polymorphic markers where different alleles are made up of different numbers of repetitive sequence elements between conserved flanking regions. On average one microsatellite is found every 100 000 bases. A complete map of microsatellite markers covering the human genome was presented by the Généthon (Dib et al, Nature, 1996, 380, 152-4). Microsatellites are genotyped by sizing PCR products generated over the repeat region on gels. The most widely used systems are based on the use of fluorescently labelled DNA and their detection in fluorescence sequencers. Fewer SNPs are in the public domain. A SNP map with 300.000 SNPs is being established by the SNP consortium *(*Science, 1999, 284, 406-407).

For genotyping SNPs, there are a few methods available for the person skilled in the art, all of them with advantages and disadvantages.

Some of these methods rely on gel-based detection, like the oligonucleotide ligase assay (OLA), and for this reason only allows medium throughput applications.

Others rely on pure hybridization which is not as discriminating and is difficult to tune to get the high stringency required (oligonucleotide arrays, DNA chips). Although DNA chips are well suited for simultaneous genotyping of a large number of genotypes in a very limited region of the genome and on an overseeable number of individuals, the main problem seen with the use of these objects is the difficulty to optimize the hybridization conditions (in particular for the stringency).

Approaches using primer extension and detection by fluorescence have been shown. Their advantage is facile emission detection in an ELISA type reader. The limitation of these methods is the limited number of fluorescent dyes available, which in return limits the number of sample that can be simultaneously analyzed.

Several methods use mass spectrometric detection, as mass spectrometry potentially allows for very high throughput and at the same time gives added information through the absolute mass. In applications where an allele specific product is measured this is direct information and therefore very strong. Nevertheless, although mass spectrometry has very high potential for genotyping of a large number of samples, the main drawback currently faced by users is the need for purifying the generated samples before the actual analysis.

In fact, a method termed the Invader assay was recently introduced (T. Griffin and L.M. Smith Proceedings of the ASMS 1998, WO98/23774, US5,843,669). For this procedure two oligonucleotides that cover a known polymorphism are applied. One oligonucleotide covers the sequence so that its 3'end is on the position of the polymorphism. Two oligonucleotides with the sequence continuing 3' of the polymorphism are used for this assay. 5' of the polymorphism they cover the different alleles and then continue with any base sequence. These two oligonucleotides are hybridized to genomic DNA. Making use of a structurally specific endonuclease the 5'overhang of the 3'standing oligonucleotide is cleaved off if there is a match with the allele of the polymorphism. The cleaved off fragment is used for characterization. This can be either by direct analysis of the cleaved off fragment by, for example, mass spectrometry or by attaching fluorescent dyes to the oligonucleotide and observing the development of fluorescence quenching. The disadvantage of this detection by mass spectrometry is that the detection sensitivity is low and that the cleaved off fragments have to be purified as the analysis of native DNA is very sensitive to impurities.

Matrix-assisted laser desorption/ionization time-of-flight mass spectrometry (MALDI) allows the mass spectrometric analysis of biomolecules (Karas, M. & Hillenkamp, F. Anal. Chem. 60, 2299-2301 (1988)). MALDI has been applied to the analysis of DNA in variations that range from the analysis of PCR products to approaches using allele specific termination to single nucleotide primer extension reactions, sequencing and hybridization (US5,885,775, WO96/29431, US5,691,141, WO97/37041, WO94/16101, WO96/27681, GB2339279).

As previously said, the major drawbacks of most of these approaches are that they heavily rely on stringent purification procedures prior to MALDI analysis that do not lend themselves to easy automation and make up a major part of the cost. Spin column purification and/or magnetic bead technology and reversed-phase purification are frequently applied.

With the future availability of the whole genome sequence and maps of SNPs being established, it will be desirable to perform SNP determination in multiple regions of the genome of an individual, in order to determine, for example, his susceptibility to a multiple genes disease. There is therefore a need to develop a method that allows the generation of a representation of the genomic DNA and the analysis of the SNP that could be present in these regions, said SNP being determined, for example by computer analysis of the regions being generated, rather than determine the SNP of interest before generating the regions containing said polymorphisms as proposed by the current methods.

Therefore, the present invention relates to a universal and generic method to solve this problem. It is also the aim of the present invention to provide a procedure for SNP analysis that is easy, cheap, highly multiplexable, easily automatable and lends itself to high-throughput.

The procedure according to the invention makes use of the potential of a highly parallel preparation of allele specific products, their conditioning so that they require no purification and the potential of mass spectrometers to distinguish large numbers of products simultaneously in one spectrum and being able to record a single spectrum in a few seconds, while achieving an appropriate signal to noise.

It is indeed difficult to match the sensitivity (signal to noise ratio) and the complexity (multiplexing) of the procedures for genotyping.

The invention provides a method for genotyping that comprises three steps:
a. reducing the complexity of the genome,
b. generating allele specific products,
c. analyzing the products by mass spectrometry.

The method of the invention is characterized in that the generation of allele specific products in step b. is achieved by at least one method that uses (an) allele specific oligonucleotide(s).

The reduction of complexity of the genome in step a. is done by a technique that leads to non-directed isolation of genomic DNA regions. It is indeed the aim of the invention to obtain a large representation of the genome and then perform the genotyping analysis for the modifications that can be present in said representation, rather than perform said analysis on a specific region of the genome that would have been pinpointed after choosing SNPs, deletions or insertions of interest.

A few methods can be alternatively used for increasing the population of defined genomic regions. The polymerase chain reaction (PCR) (Mullis et al., (1986) Cold Spring Harbor Sym. Quant. Biol., 51, 263-273) amplifies a stretch of DNA sequence by making use of two oligonucleotide recognition sequences (primers), a DNA polymerase and constituent DNA building blocks. Usually, the primers are defined in order to amplify a selected DNA region. The PCR shall be used, in accordance to the present invention, to amplify significant parts of a genome rather than short and specific parts.

There are two concepts for the amplification of defined and significant parts of a genome. DOP-PCR (degenerate oligonucleotide primer PCR) uses primers that have one or several degenerate bases in them. Through this, fair distribution of representation across the genome can be achieved. If two of the degenerate sequences face each other in a close enough range for the chosen PCR conditions, a PCR product is generated (Telenius et al., Genomics, 1992, 13, 718-25).

The second method is ALU PCR. For this heavily represented, repetitive sequences of the genome are used as primers. In this case sequences of ALU repeats are used (Nelson et al., Proc. Natl. Acad. Sci. USA, 1989, 86, 6686-90).

These two methods can be combined with Long Range PCR, that allows amplification of very large sequences of DNA.

The ligase chain reaction (LCR) uses four oligonucleotide recognition sequences (oligonucleotides) and a DNA ligase (Landegren et al., Science, 1988, 241, 1077-80).

Another possibility for the generation of large regions of DNA is the production of padlock probes. This method uses linear pieces of DNA circularized by template directed ligation and can be followed by rolling circle amplification of these templates (WO99/49079, WO97/09069)

Another method for the reduction of the complexity of the genome is amplified restriction length polymorphism (AFLP). For AFLP, the genomic DNA is digested with a restriction enzyme. Oligonucleotides with known sequences are ligated to the ends of the restriction fragments. These bound sequences together with the first few bases of the ligated product are used as primer sequences for a PCR. The choice of the few bases of the primer allows the selection and the extraction of only a fraction of the genomic sequence (Vos et al., Nucleic Acid Research, 1995, 23, 4407-14).

Another method that could be used for the reduction of the complexity of the genome is the cleavage reaction. In this implementation, the reduction of complexity is achieved by cleaving off known sequences of oligonucleotides. The known cleavage products that can be generated in large numbers are a reduced complexity representation of the genomic DNA (Griffin et al., 1999, Proc. Natl. Acad. Sci. USA, 96, 6301-6).

Sauer et al. Nucleic Acids Research 2000 Vol. 28 No. 5 e13 describe a method to genotype SNPs involving PCR, allele specific primer extension and MALDI mass spectrometry.

As previously said, after reduction of the complexity of the genome, the method used being chosen by the person skilled in the art in function of the aimed goal, the SNP that will be detected can preferably be chosen by computer-assisted analysis, by using data available in the public databases.

The generation of the allele specific products is performed by using allele specific oligonucleotides. These oligonucleotides usually harbor a degenerate base that is specific for onr of the alleles of the SNP that is to be tested, and correctly interacts only with that one allele of the SNP.

To detect the complete hybridization, it is necessary to perform a few steps after hybridization of the allele specific oligonucleotides. The method that can be used are primer extension, allele specific ligation, or cleavage reaction.

Primer extension is done with allele specific primers and a DNA polymerase.

Preferably, the oligonucleotide is about 17-25 bases long and chimeric in nature. One part (the 5'end) is regular DNA with phosphodiester bonds, while modifications are introduced near the 3'end of the oligonucleotide. These later serve to enhance the detection sensitivity in the mass spectrometer. In the most preferred embodiment, the oligonucleotides carry modifications that allow separation of the two different parts of the chimeric oligonucleotide either by chemical or enzymatic cleavage.

As well, allele specific ligation uses an oligonucleotide that is allele specific (the 3' end of which being the SNP) and another oligonucleotide that is immediately 3' from the first one. The ligation will only be performed if the two oligonucleotides hybridize the template at the site of the ligation (Landegren et al., Science, 1988, 241, 1077-80). Alternatively the allele specificity can be achieved by matching the 5' end base of the 3' standing oligonucleotide.

Preferably the oligonucleotides are chimeric in nature. The 5' standing oligonucleotide carries its modification on its 3' end while the 3' standing oligonucleotide carries its modification on its 5' end. These later serve to enhance the detection sensitivity in the mass spectrometer. In the most preferred embodiment, the oligonucleotides carry modifications that allow separation of the two different parts of the chimeric oligonucleotide either by chemical or enzymatic cleavage.

In another implementation the allele specific products are generated by cleavage of an overhang by a cleavase, a flap-endonuclease (FEN), a resolvase or an endonuclease. One oligonucleotide covers the sequence immediately 5' up to the polymorphism. A second oligonucleotide, that is composed that it completely covers the sequence up to the first oligonucleotide for one allele of the polymorphism but not for the other is added to the preparation. This oligonucleotide is of chimeric nature and has a 5'overhang, that is modified.

Further the preparation is incubated with a structurally specific endonuclease. This endonuclease cleaves off the overhanging part of the 3'standing oligonucleotide that sticks away from the DNA double strand in the case of a complete match - one allele of the polymorphism. This part is then used for analysis by mass spectrometry. Two different oligonucleotides that cover the two alleles of a polymorphism can be used simultaneously. The 5'end of the two chimeric oligonucleotides can be chosen so that each cleaved off product has a different mass. Alleles are assigned by the presence or absence of the different masses. The nature of the cleaved off fragment is chosen so that it can be detected by mass spectrometry with high sensitivity and without purification directly from the reaction mixture.

It has to be understood that the reduction of the complexity of the genome can be performed by using more than one of the methods described above. It would therefore lead to a larger representation of the genome than by using only one method. Depending on the SNP to be analyzed, one could also combine and use several methods, at least one of which using allele specific oligonucleotide(s). the person skilled in the art can combine two or more methods depending on the allele specific products to be used (number, sequences...).

The method of the invention shows the combination of different means of reducing the genome complexity with allele specific sample preparation techniques. In fact, none of the previously described procedures for generating allele specific products can be applied directly to genomic DNA.

On the other hand the detection sensitivity of the mass spectrometer in combination with the applied charge tag technology is sufficient to detect the products generated by the described methods for allele specific sample preparation directly from genomic DNA. Due to the representation of most 20-mer base recognition sequences in genomic DNA of, for example humans, insufficient signal to noise is achieved and therefore genotyping is not possible.

The present invention allows to solve these problems by providing a general strategy that can be used for genotyping multiple SNPs in one series of experiments. One of the strengths of this invention is that it provides possibilities to combine any reduction of complexity method with any allele specific product generation and that the products are easily immediately available for mass spectrometric analysis.

As the analysis of nucleic acids by MALDI is strongly dependent on the charge state of the molecule to be tested, a 100-fold increase in analysis sensitivity can be achieved when the DNA is conditioned to carry one positive charge. Such modified DNA products are also significantly less susceptible to adduct formation and so do not require purification procedures (Gut and Beck (1995) Nucleic Acids Res., 23, 1367-1373; Gut et al., Rapid Commun. Mass Spectrom., 11, 43-50 (1997)).

It is advantageous to use modified oligonucleotide(s) in the step b. of the method of the invention as to allow a mass spectrometric analysis with significantly higher sensitivity than for (an) unmodified and unpurified oligonucleotide(s).

It is also important to condition the products generated in step b. of the method of the invention to carry a single excess charge, either positive or negative. It is usually possible to modify said products generated in step b. to carry a single charge.

To achieve this goal, the oligonucleotides can be chosen so that they can be cleaved off after generation of the allele specific products, the cleaved product fulfilling the requirement of bearing a single charge or being able to be chemically modified to get to this charge state.

In one preferred implementation of this invention the cleaved off part is a peptide nucleic acid, a peptide, a methylphosphonate, ethylphosphonate, a methoxyphosphonate, or an ethoxyphosphonate. In another preferred implementation the cleaved off part is an oligonucleotide containing phosphorothioate linkages. A single positive charge can be introduced into the cleavage product by coupling a charge containing functionality to it. This can be achieved by condensation of a positive charge tag function to the 5'end of the overhang by an amino function, by attaching it to one of the bases of the overhang or by leaving a regular phosphate group with the cleaved off part of the oligonucleotide and chosing the backbone of the overhang such that it can be charge neutralised by alkylation of for example phosphorothioate bridges or by choosing an oligonucleotide modification that is already uncharged.

It is therefore important to use chimeric oligonucleotides, consisting of a regular sugar phosphate (phosphodiester bonds) backbone, with an end (most preferably the 5' end) being a phosphorothioate, ethylphosphonate, methoxyphosphonate, ethoxyphosphonate, phosphoroselenoate, peptide nucleic acid, or a peptide. Preferably, the oligonucleotides are between 13 and 40 bases long.

It is also preferred that the unmodified part of the oligonucleotide(s) is separated from the modified part, after generation of the allele specific products. This can be performed by using chemical cleavage or enzymatic cleavage. The enzymatic cleavage reaction is preferably an inhibited nuclease digest. The reaction of cleavage with the endonuclease or exonuclease is inhibited as not to digest the products that are to be analyzed.

The modification of the oligonculeotides is important as it allows a mass spectrometric analysis with higher sensitivity than for an unmodified oligonucleotide. Indeed, due to the large number of negative charges in native DNA, it is often difficult to analyze DNA by mass spectrometry. The method of the invention uses chimeric oligonucleotides to solve this problem and allow the easy detection of numerous SNPs at the same time.

For example, when the oligonucleotides are partly phosphodiester and partly phosphorothioate, the part that is cleaved off contains the phosphorothioate bonds. Charge neutralization of the phosphorothioate bonds can be easily achieved by alkylation.

Charge tagging of the cleavage products will be performed by two slightly different strategies depending on the used ion modes (positive and negative):
- for positive charge tagging a base that contains either a positive charge functionality or at least a chemical function that later allows the introduction of a charge tag into the part of the oligonucleotide that is later allele specifically cleaved off.
- for charge tagging for negative ion mode the cleaved off part can be synthesized so that a single phosphate group remains in the cleaved off fragment while the rest are phosphorothioates. As the alkylating reaction is selective for the phosphorothioate groups, the phosphate group remains unchanged and thus acts as the negative charge tag. In a preferred embodiment of the invention the phosphate group is closest to the cleavage site of the structurally active endonuclease. This secures optimal operation of the endonuclease

In another embodiment, the oligonucleotides are modified to be partly phosphodiester DNA, partly PNA. The cleavage position is a regular sugar-phosphate bond that is cleaved by the structurally sensitive endonuclease. The chimeric oligonucleotide can be synthesized so that a residual phosphate bond remains with the cleavage product. The negative charge of the phosphate group can serve as the charge required for the extraction of the product in the mass spectrometer. Such oligonucleotides can be used in the cleavage procedure for the generation of allele specific products, in that the PNA achieve high sensitivity in mass spectrometric analysis and do not require purification prior to analysis.

This is another advantage of the procedure of the invention, as the chemical modifications foreseen in the allele specific oligonucleotides used for the generation of the allele specific products allow the analysis of said products by mass spectrometry without purification or separation from the reaction mixture.

As the method of the invention is intended to be used for the analysis of a lot of SNPs at the same time, it is important that each allele specific product generated in step b. harbors a unique mass that allows unambiguous allele assignment of said product. This can be achieved by carefully choosing the allele specific oligonucleotides and the method for the generation of allele specific products. A computer-assisted analysis will help the person skilled in the art in determining the effective oligonucleotides to use. It is usually best to use oligonucleotides designed in a way that the products generated in step b. are between 2 and 15, more preferably between 2 and 10 bases long.

The analysis of the products generated by the chosen allele specific method(s) is performed by mass spectrometry. Although MALDI is a preferred method, another embodiment of the procedure uses electrospray ionization mass spectrometry, as described in the patent application WO 99/29897.

When the MALDI is used, the choice of the matrix can be important for the ionization of the allele specific products. One could use a matrix with good ionizing properties, or with weakly ionizing properties. One could also use a matrix which exhibits a strong absorption at the laser wavelength.

One would preferably use a matrix such as α-cyano-4-hydroxycinnammic acid, α-cyano-4-hydroxycinnamic acid methyl ester, α-cyano-4-methoxycinnamic acid matrices, derivatives thereof, and a mixture of these matrices.

The conditions of the mass spectrometry analysis will also be determined by the person skilled in the art to allow the analysis of the modified fragments obtained from the allele specific products, while all reactions by-products (including the regular phosphodiester backbones that have been cleaved off) are not detectable. The choice of the matrix is very important for such a specific detection of the desired products.

The method described in the present invention is therefore an universal method that allows the genotyping of multiple SNPs from genomic DNA of an individual. After reduction of the complexity of the genome, the DNA is genotyped by using allele specific oligonucleotides that are preferably modified, in order to allow their analysis by mass spectrometry without purification. The method shows a big improvement as compared to the previous art in that it is the first described procedure that can really allow to use the full potential of the mass spectrometric detection in genotyping, principally multiplexing and automation which was previously hampered by the need to purify products.

The following examples illustrate different embodiments of the invention, and the operating conditions described in said examples should not be considered as limiting the invention, and can be optimized by the person skilled in the art for each application.

### DESCRIPTION OF THE FIGURES

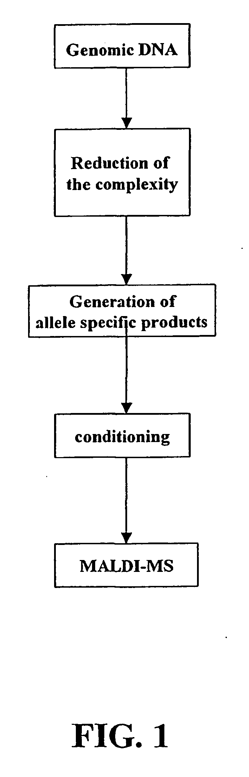

Figure 1: Principle of the invention.

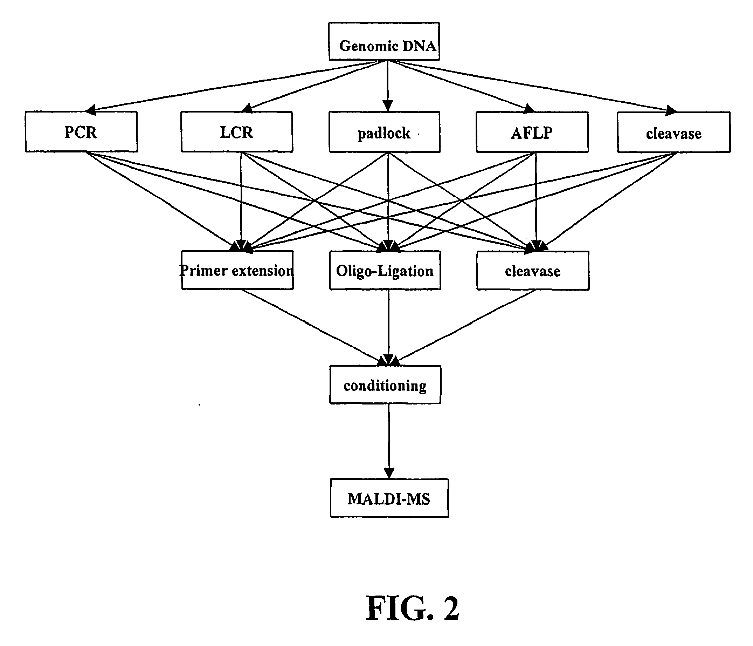

Figure 2: Detailed overview of the invention. The complexity of the genomic DNA is reduced, and allele specific products are generated, that can be optionally conditioned by simple modification chemistry, and are analyzed by mass spectrometry and without the need for purification.

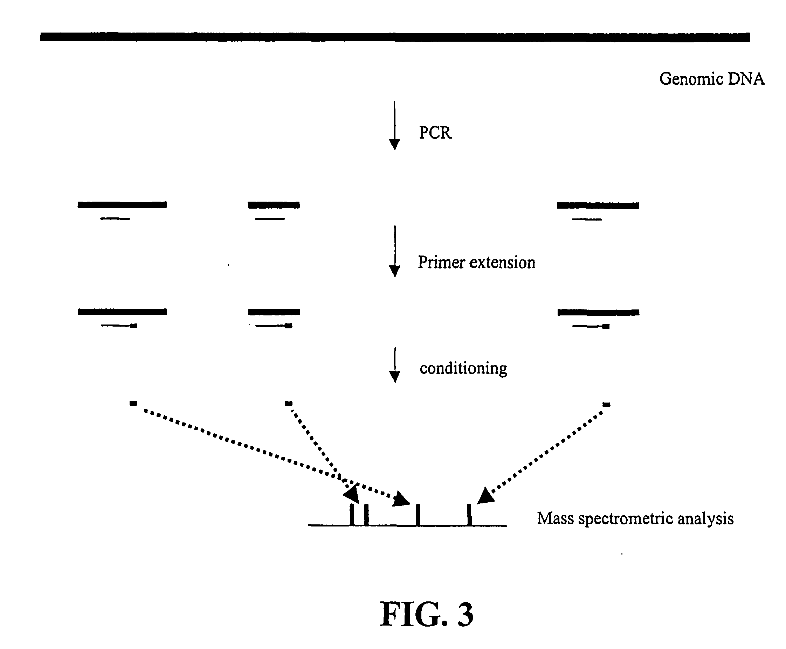

Figure 3: PCR complexity reduction and primer extension. The reduction of the complexity is done by the use of one of the described PCR methods. Afterwards the resulting amplification products are used as templates for an allele specific primer extension reaction. The unmodified parts of the primer extension products have to be removed and the resulting product are analyzed by MALDI.

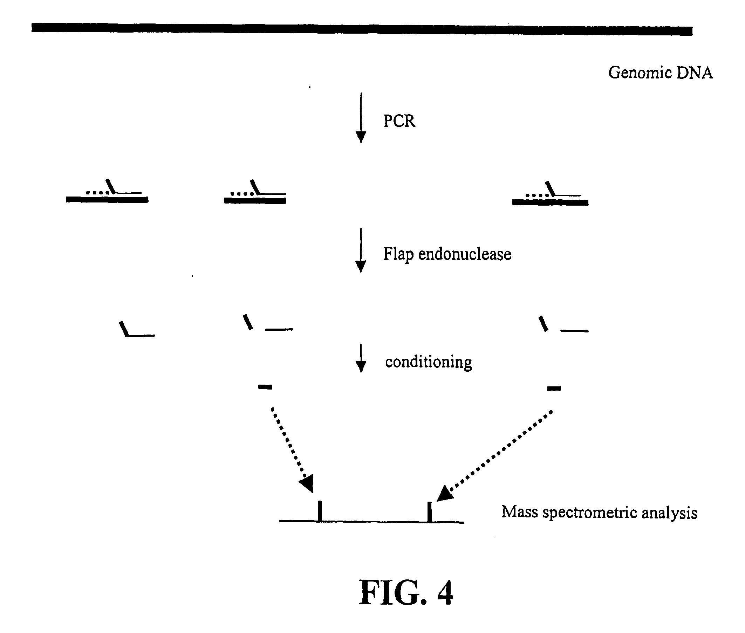

Figure 4: PCR complexity reduction and flap endonuclease. The reduction of the complexity of the genomic DNA is achieved by the use of a flap endonuclease reaction. The allele specific product generation is also executed by a flap endonuclease reaction. The conditioning of the products for the MALDI analysis is performed as described in the specification.

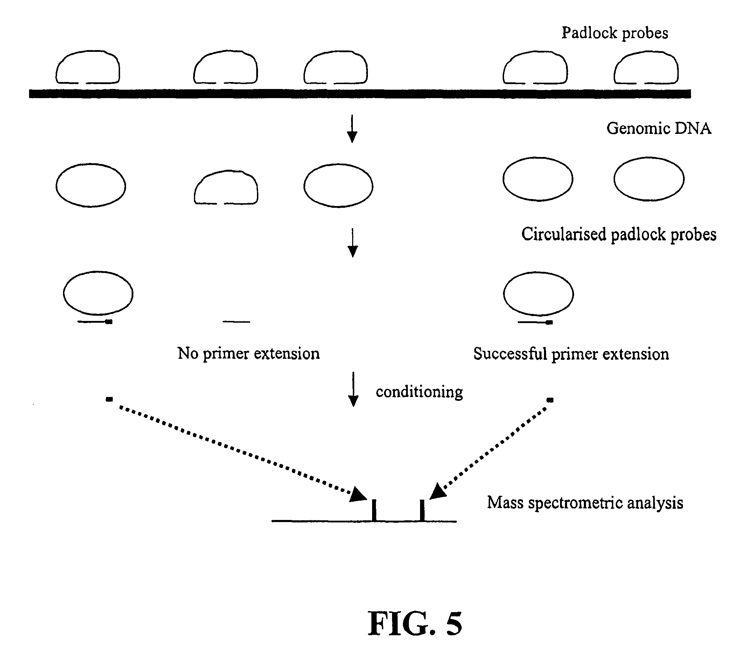

Figure 5: Padlock complexity reduction and primer extension. The reduction of the complexity of the genomic DNA is achieved by the use of several padlock probes.
The circularized products can be used for a rolling circle amplification. The resulting products serve as templates for an allele specific primer extension reaction, as already described.

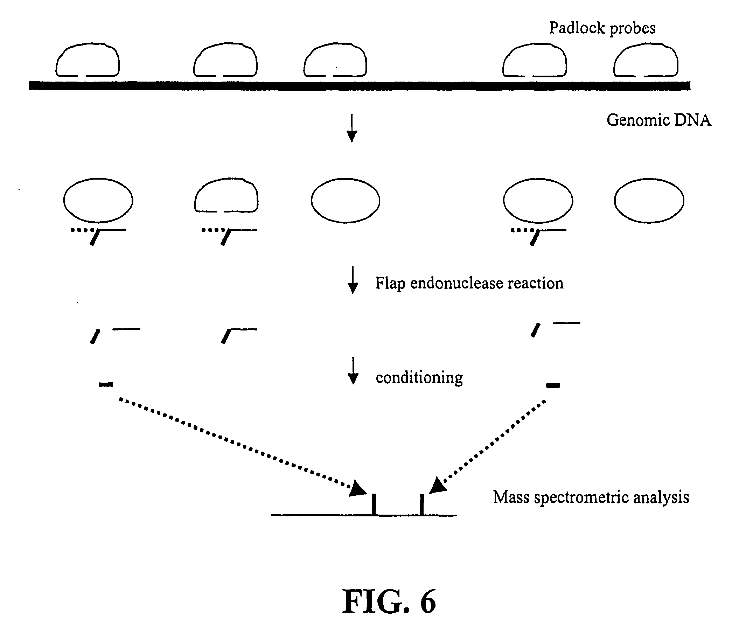

Figure 6: Padlock complexity reduction and flap endonuclease. The reduction of the complexity is performed by generation of padlock probes. In this case the generation of allele specific products is achieved by a Flap endonuclease reaction.
The rest has to be done as already described.

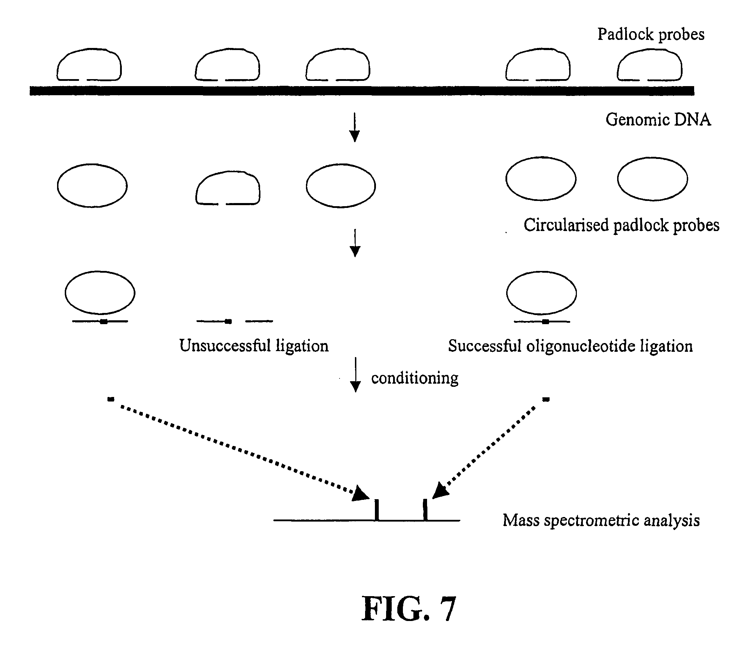

Figure 7: Padlock complexity reduction and oligonucleotide ligation. The reduction of the complexity of the genomic DNA is achieved by generation of padlock probes. The allele specific product generation is executed by an oligonucleotide ligation assay. The rest is done as described.

### EXAMPLES

### Example 1: PCR and oligonucleotide ligation

PCR: 40 mM Tris-HCl, 32 mM (NH₄)₂SO₄, 50 mM KCI, 2 mM MgCl₂ at pH 8,8, 5 pmoles of forward and reverse primers, 200 µM dNTPs, 5 ng genomic DNA and 0,2 U Taq-DNA-Polymerase are filled up with water to a 10 µl reaction volume. The reaction is denatured 2 min at 95 °C, then thermocycled 20 s at 95°C, 30 s at the appropriate annealing temperature (e.g. 65°C) and 30 s at 72 °C 30 times.

Ligation: 5 µl of the PCR are taken for the ligation step. Three oligonucleotides (each 25 pmole) are used for the ligation. Two of them contain a allele specific sequence and one is employed as the ligation partner. The 3'-oligonucleotide carries a phosphate group at its 5'-end and a backbone modification at its bridge between nucleoside one and two (and two and three and so on) from the 5'-end that can be a phosphorothioate, a methylphosphonate, a peptide nucleic acid or similar. The 5'-oligonucleotide contains a backbone modification such as the mentioned examples at its 3'-end. 5 U of a (thermostable) Tth DNA ligase and 20 mM Tris-HCl, 20 mM KCl, 10 mM MgCl₂, 0,1 % Triton X-100, 0,1 mM NAD, 10 mM DTT at pH 7,5 are filled up with water to a 20 µl reaction volume. The reaction is denatured 2 min at 95 °C, then thermocycled 15 s at 95 °C and 90 s at the appropriate ligation temperature (e.g. 45 °C) 25 times.

Exonuclease digestion: A 5'- and a 3'-exonuclease are used for the digestion of the bulk of (unmodified) oligonucleotides. The pH of the reaction has to be roughly adjusted by acetic acid to pH 7. As a 5'-exonuclease 2 U of 5'-phosphodiesterase from calf spleen and 10 U of the 3'-exonuclease Exo III are incubated for 1 h at 37 °C. This procedure can also be performed in a two-step process by using first 5 U of 3'-phosphodiesterase from snake venom and incubation for one h at 37°C. In a second step the pH is adjusted to pH 7 and 2 U of 5'-phosphodiesterase from calf spleen are incubated four 1 h at 37 °C.

The phosphorothioate containing products are alkylated in 45 µl acetonitrile, 15 µl triethylammoniumbicarbonat buffer (pH 8,5) and 14 µl methyliodide for 25 min at 40 °C. Then 20 µl water are added and 20 µl of the upper phase are mixed with 45 µl of 40 % acetonitrile.

The methylphosphonate containing products are diluted in 50 to 250 µl of 40 % acetonitrile.

Afterwards 0,4 µl of the products are loaded on a target coated with a thin layer matrix such as α-cyano-4-hydroxycinnamic acid methyl ester.

(Sauer et al. Nucleic Acids Res. 28, e13 (2000), Landegren et al. Science 241, 1077-1080 (1988)).

### Example 2: One-step PCR and ligation

The PCR and the ligation are executed in one step. 10 mM Tris-HCl, 50 mM KCl, 1mM NAD, 200 µM dNTPs, 6 mM MgCl₂, 2,5 µM of each PCR primer (25-30-mers) and 0,2 µM of each ligation oligonucleotide (12-17-mers), 5 ng genomic DNA, 0,2 U of Taq-DNA-Polymerase and 5 U of a (thermostable) DNA ligase are filled up with water to a 20 µl reaction volume. The reaction is denatured 2 min at 95 °C, then thermocycled 15 s at 95 °C, ramping slowly to 65 °C over 90 s, 30 s at 65 °C 10 times, 15 s at 95 °C and 30 s at 65 °C 30 times, 15 s at 95 °C and 90 s at 45 °C 25 times.

The following steps are executed as in example 1.

### Example 3: Ligase chain reaction and oligonucleotide ligation

Reduced complexity templates are generated by using a set of six ligation oligonucleotides. Three of them work in such a way as in the first and second example but on genomic DNA and not on PCR products. The generated products serve as templates for the other set of three oligonucleotides. The result of this approach is an efficient product amplification.

50 mM N-(2-hydroxyethyl) piperazine-N-(3 propanesulfonic acid), 10 mM MgCl₂, 10 mM NH₄Cl, 80 mM KCI, 1 mM DTT, 1 µg BSA per ml, 100 µM NAD and 1 pmol of each of the six oligonucleotides (e.g. 20-mers) within a set and 5 U of a thermostable DNA ligase are filled up with water to a 50 µl reaction volume. The reaction is denatured for 2 min at 95 °C, then thermocycled 20 s at 95 °C, 90 s at the appropriate annealing and ligation temperature (e.g. 55 °C) 30 times.

(Nickerson et al, Proc. Natl. Acad. Sci. USA 87, 8923-8927 (1991), Baron et al. Nature Biotechnology 14, 1279-1282 (1996)).

The following steps are done the same as in example 1.

The principle of the variant using ligation is shown for the single nucleotide polymorphism 61 of the human gene for granulocyte-macrophage colony stimulating factor (GM-CSF).

Template:
5'-TTACTGGACTGAGGTTGC[G/A]CCTGCTCCAGGGAGCCCATGTGAC-3' (SEQ ID N° 1 and 2)
3'-AATGACCTGACTCCAACG[**G/T**]* **GG**ACGAGGTCCCTCGGGTA-5' (SEQ ID N° 3 and 4).
* the single nucleotide polymorphism in this example is detected by a ligation of two allele specific oligonucleotides that are selectively connected. The 3'-oligonucleotide contains a free phosphate group at its 5'-end, the 5'-oligonucleotide contains a free hydroxyl group at its 3'-end.

The sequences in bold underlined show the allele specific products which are generated after the 5'- and 3'-exonuclease digestion. The digestion is inhibited by the appropriate modification of the phosphate backbone of the oligonucleotides. In the case of phosphorothioates the backbone must be charge neutralized by alkylation. This step can be made superfluous by the use of e.g. methylphosphonates. The products have to be prepared for the MALDI process as described above.

In order to improve the amount of products and to circumvent a PCR amplification step the product of the first ligation could serve as a new template for another set of allele specific products which is shown below.
2. product:
   5'-TACTGGACTGAGGTTG**C[C/A]*CC**TGCTCCAGGGAGCCCATG-3' (SEQ ID N° 5 and 6).
1. product:
   3'-AATGACCTGACTCCAACG[G/T]GGACGAGGTCCCTCGGGTA-5' (SEQ ID N° 3 and 4).

### Example 4: Ligase Chain Reaction and primer extension

In order to improve the selectivity of the approach which is described in the third example, a primer extension step is used following the LCR that was done with the conditions described in example 2.

Primer extension reaction: 25 pmoles of the charge tagged primer was added together with 100 µM α-S-dNTPs (reduced set, at least one of the four bases is omitted and in some cases replaced with the corresponding α-S-ddNTP) and 0.5 U of Thermosequenase. The reaction volume was increased to 20 µl by the addition of water. An initial denaturing step 3 min at 95°C was used followed by 40 cycles of 20 s at 95°C, 1 min at 58°C and 1 min at 62°C. Primer Removal: 1 µl of a 0.5 M acetic acid solution was added to the processed extension reaction resulting in a reaction pH < 7.0. Then 2 µl of 5'-phosphodiesterase, that was previously dialysed against ammonium citrate (0,1 M, pH 6,0) was added and the reaction incubated for 45 min at 37°C.

Alkylation reaction: 45 µl of acetonitrile, 15 µl of triethylammonium bicarbonate solution (pH 8,5) and 14 µl of CH₃I were added. The reaction was incubated at 40 °C for 25 min. Upon cooling a biphasic system was obtained. 20 µl of the upper layer was sampled and diluted in 45 µl of 40 % acetonitrile. This solution was directly used to transfer the samples onto the matrix.

Sample preparation for MALDI analysis: The α-cyano-4-hydroxy-cinnamic acid methyl ester (CNME) matrix was prepared by spotting 0.5 µl of a 1% solution in acetone onto the target and spotting 0.3 µl of a solution of the sample in 40 % acetonitrile on top of the dried matrix. 40 % acetonitrile dissolves the surface layer of the matrix allowing a concentrated incorporation of the analytes into the matrix surface. By this preparation, a very thin and fine crystalline matrix layer was achieved. CNME was synthesized according to the method of Gut et al. (Rapid Communications in Mass Spectrometry 11, 43-50 (1997)).

### Example 5: Reduction of genome complexity by a flap endonuclease

Flap endonuclease reaction: 1 µl reaction buffer (16 % polyethylene glycol, 50 mM (3-[N-Morpholino] propanesulfonic acid, pH 7,5), 1 µl of 12 µM primary oligonucleotide, 1 µl of 500 ng/µl human genomic DNA or RNA or a comparable amount of a (RT-) PCR product and 3 µl water are mixed together. The mix is incubated for 5 minutes at 95 °C for DNA denaturation. Then the reaction mix is cooled down to 63 °C and 3 µl of a solution containing 75 mM of MgCl₂ and 5 pmol of each of the two primary probe oligonucleotides and 100 ng of a flap-endonuclease (e.g. Afu FEN 1 from *Archaeoglobus fulgidus* or Pfu FEN 1 from Pyrococcus furiosus) are added. The reaction mix is incubated for 2 hours at 63°C. The fragments cleaved off secondary oligonucleotides serve as a reduced complexity representation of the DNA that is to be queried. The cleaved off fragments can be used as new primary oligonucleotides for a successive flap endonuclease reaction on a synthetic template that is provided at a significantly higher concentration as the genomic DNA. Due to the duty cycle of the primary reaction a substantial representation for the subsequent allele specific reaction is provided as is shown in example 6 for example by a highly multiplexed reaction with several probe oligonucleotides and intruder oligonucleotides for several loci (Kaiser et al. The Journal of Biological Chemistry 30, 21387-21394 (1999), Lyamichev et al. Nature Biotechnology 17, 292-296 (1999), Griffin et al. Proc. Natl. Acad. Sci. USA 96, 6301-6306 (1999)).

### Example 6: Flap endonuclease reactions for the generation of allele specific products

Flap endonuclease reaction: 1 µl reaction buffer (16 % polyethylene glycol, 50 mM (3-[N-Morpholino] propanesulfonic acid, pH 7,5), 1 µl of 12 µM primary oligonucleotide, 1 µl of 500 ng/µl human genomic DNA or RNA or a comparable amount of a (RT-) PCR product and 3 µl water are mixed together. The mix is incubated for 5 minutes at 95 °C for DNA denaturation. Then the reaction mix is cooled down to 63 °C and 3 µl of a solution containing 75 mM of MgCl₂ and 5 pmol of each of the two primary probe oligonucleotides and 100 ng of a flap-endonuclease (e.g. Afu FEN 1 from *Archaeoglobus fulgidus* or Pfu FEN 1 from *Pyrococcus furiosus*) are added. The reaction mix is incubated for 2 hours at 63°C.

The cleaved product from the probe can contain already charge neutral backbones such as methylphosphonates or modifications that can be made charge-neutral such as phosphorothioates by an alkylation procedure. The charge-tag can be either positive (e.g. via an aminomodification) or negative (e.g. via a phosphate bridge).

Used oligonucleotides for the SNP 22708 (A/T) in the angiotensin converting enzyme gene (ACE). The methylphosphonates ("mp") can be replaced by phosphorothioates, or other modified nucleotides. The cleavage side of the probes is between position 4 and 5 from the 5' end:
Meddler/Intruder ACE 22708 1: SEQ ID N° 7
Probe 1 ACE/MPPos (SEQ ID N° 8):
5'-**T(NH₂)ₘₚTₘₚAₘₚ****A**GGGCAATACAGCAAGACCCCGTCT
Probe 2 ACE/MPPos (SEQ ID N° 9)
5'-**T(NH₂)ₘₚTₘₚAₘₚ****T**GGGCAATACAGCAAGACCCCGTCT

Probe 1 ACE/MPNeg (SEQ ID N° 10): 5'-**TₘₚTₘₚAA**GGGCAATACAGCAAGACCCCGTCT
Probe 2 ACE/MPNeg (SEQ ID N° 11): 5'-**TₘₚTₘₚAT**GGGCAATACAGCAAGACCCCGTCT

The underlined bases correspond to methylphosphonate bases. The first T at the 5' end of these probes bears a NH₂ moiety that allows to positively charge the obtained fragments (in bold).

The products (in bold) have to be prepared for the MALDI process as described in the ligation section via exonuclease digestion and charge neutralisation.

In contrast to the previous example the used probe oligonucleotides can be unmodified but it is absolutely necessary that the cleaved product is longer than in the first example because it should serve as a new intruder oligonucleotide for the second target.
Probe 1 ACE/IN (SEQ ID N° 12): 5'- GGCCCTGTAACTCGGAA GGGCAATACAGCAAGACCCCGTCT
Probe 2 ACE/IN (SEQ ID N° 13): 5'- GGAACTGTGGCTCTTAT GGGCAATACAGCAAGACCCCGTCT

Intruder oligonucleotide that derives from Probe 1 ACE/IN (SEQ ID N° 14): 5'-GGCCCTGTAACTCGGAG
Intruder oligonucleotide that derives from Probe 2 ACE/IN (SEQ ID N° 15): 5'-GGAACTGTGGCTCTTAA

Second synthetic templates:
1. SEQ ID N° 16
2. SEQ ID N°17

Probe sq. 1 pos (SEQ ID N° 18): 5'-**C(NH₂)ₘₚTₘₚGₘₚT**GGACCGTCGTACACCG
Probe sq. 2 pos (SEQ ID N° 19): 5'-**G(NH₂)ₘₚCₘₚTₘₚGₘₚ****A**GCAGTGTCGTACATTG

The underlined bases correspond to methylphosphonate bases. The first C and the first G at the 5' end of these two probes bears a NH₂ moiety that allows to positively charge the obtained fragments (in bold).
Probe sq. I neg (SEQ ID N° 20): 5'-**CₘₚTₘₚ****GT**GGACCGTCGTACACCG
Probe sq. 2 neg (SEQ ID N° 21): 5'-**GₘₚCₘₚTₘₚ**GAGCAGTGTCGTACATTG

The underlined bases correspond to methylphosphonate bases. The first T at the 5' end of these probes bears a NH₂ moiety that allows to positively charge the obtained fragments (in bold).

The products (in bold) have to be prepared for the MALDI process as described.

### Example 7: Padlock probe ligation

The probe (SEQ ID N° 22) that is chemically 5'-phosphorylated and reversed-phase purified has a concentration in a range of 0,01 µM. In the part of the probe indicated as N₄₀ a primer sequence for the following RCA reaction and also a restriction site for *Hpa*II is contained. 50 ng genomic DNA are used as template. 50 mM KAc, 10 mM MgAc₂, 10 mM Tris-Ac (pH 7,5), 1 mM ATP and 0,1 µg/µl bovine serum albumin (BSA) in a volume of 20 µl are heated to 65 °C and cooled to room temperature. Then 1 µl of a T 4 DNA ligase (0,5 U/µl) is added and incubated at 37 °C for one hour. The ligation is terminated by incubation at 65 °C for 10 minutes.

RCR reaction: 5 µl of the ligation reaction are used and filled up with 50 mM Tris-HCl (pH 7,5), 10 mM MgCl₂, 20 mM (NH₄)₂SO₄, 1mM dithiothreitol and 0,2 ug/ul BSA, 0,25 mM dNTPs, 0,1 pmol primer and 2 ng/ul phi DNA polymerase to a volume of 20 µl. The reaction is heated to 65 °C and cooled to room temperature prior to addition of the polymerase.

The RCR products are restriction digested by adding an oligonucleotide complementary to the *Hpa*II restriction site integrated in the rolling circle amplified probes to the reaction and adjusting the buffer to conditions recommended by the manufacturer. The reactions are heated to 65 °C and cooled to room temperature. Then 10 U of *Hpa*II are added. The digestion is incubated at 37°C for 12 hours and stopped by incubation at 65 °C for 10 minutes.

The sequences in N₄₀ must contain a sequence for the primer for rolling circle amplification, for example SEQ ID N° 23. This oligonucleotide contains a *Hpa*II restriction site (CCGG).

The oligonucleotide that is necessary for the *Hpa*II restriction is complementary to the primer for rolling circle amplification and contains the respective *Hpa*II restriction site (CCGG) (Lizardi et al. Nature Genetics 19, 225-232 (1998), Banér et al. Nucleic Acids Research 26, 5073-5078 (1998)).

### Example 8: Degenerate oligonucleotide (DOP) and Alu PCR

The primers that are used are SEQ ID N° 24 in case of a degenerate PCR, or SEQ ID N° 25 that represents a certain Alu sequence for Alu PCR. 2 mM MgCl₂, 10 mM Tris-HCl , pH 8,4, 50 mM KCI, 2 µM primer, 0,2 mM dNTPs, 0,01 % gelatin, 1,5 U Taq DNA Polymerase and 500 ng DNA in a 50 µl volume. Cycling procedure: Initial denaturation at 94 °C for 4 minutes, then 35 cycles: 94 °C for 1 minute, 55 °C. for 50 seconds, 70 °C for 7 minutes. (Tagle and Collins Nucleic Acids Research 1, 1211-122 (1992), Telenius et al. Genomics 13, 718-725 (1992)).

### Example 9: Arbitrary PCR

### Primer sequence: SEQ ID N° 26

40 mM Tris-HCl, 32 mM (NH₄)₂SO₄, 50 mM KCI, 6 mM MgCl₂ at pH 8,8, 0,5 µM primer, 600 µM dNTPs, 0,01 % gelatin, 10 to 25 ng DNA genomic DNA and 2 U Taq DNA polymerase are filled up with water to a 10 µl reaction volume. Thermal cycling is executed for 45 cycles of 1 minute at 94 °C, 1 minute at 45 °C and 2 minutes at 72°C. (Welsh et al. Nucleic Acids Research 19, 303-306 (1991)).

### Example 10: Amplified fragment length polymorphism (AFLP)

Modification of DNA and template preparation: The AFLP oligonucleotides consist of a core sequence, a restriction enzyme specific sequence and a selective extension site. 0,5 µg genomic DNA are incubated for 1 hour at 37 °C with 6 U EcoRI and 6 U MseI in 40 µl 10 mM Tris-HAc, ph 7,5, 10 mM MgCl₂, 50 mM KAc, 5 mM DTT, 50 ng/µl BSA. Afterwards 10 µl of a solution containing 5 pmol *Eco*RI adapters (SEQ ID N° 27 and SEQ ID N° 28), 5 pmol *Mse*I adapters (SEQ ID N° 29 and SEQ ID N° 30), 1 U T4 DNA ligase, 1 mM ATP in 10 mM Tris-HAC, pH 7,5, 10 mM MgAc, 50 mM KAc, 5 mM DTT, 50 ng/µl BSA are added and incubated for 3 hours at 37 °C. After ligation, the reaction mixture is diluted to 500 µl with 10 mM Tris-HCl, 0,1 mM EDTA, pH 8,0 and frozen.

General procedure for AFLP reactions: An *Eco*RI and a *Mse*I containing restriction site primers are used in this example SEQ ID N° 31 for *Eco*RI and SEQ ID N° 32 for *Mse*I*.*

20 µl containing 5 ng EcoRI primer and 30 ng MseI primer, 5 µl template DNA, 0,5 U Taq DNA polymerase, 10 mM Tris-HCl, pH 8,3, 1,5 mM MgCl₂, 50 mM KCl and 200 µM dNTPs. The cycling scheme depends on the different extensions of the AFLP primers. Reactions having none or a single nucleotide are performed for 20 cycles with the following profile: 30 seconds at 94 °C, 1 minute at 56 °C and 1 minute at 72 °C. Reactions with primers having two or three selective nucleotides are performed for 35 cycles: 30 seconds at 94 °C, 30 seconds annealing (see comment below) and 1 minute at 72 °C. The annealing temperature is chosen at 65 °C for the first cycle and reduced each cycle by 0,7 °C for the next 12 cycles and is continued for the remaining cycles at 56 °C.

It is recommended to preamplify with two AFLP primers having only one selective nucleotide. In this case 30 ng of both primers are used. After preamplification the reaction is diluted 10-fold with 10 mM Tris-HCl, 0,1 mM EDTA, pH 8,0. A second amplification round has to be executed as described above with AFLP primers containing longer selective extensions (Vos. et al. AFLP: a new technique for DNA fingerprinting. Nucleic Acids Research 23, 4407-4414 (1995)).

### Example 11 : Allele specific primer extension

Primer extension with allele specific primers for the single nucleotide polymorphism 61 of the human gene for granulocyte-macrophage colony stimulating factor (GM-CSF) (see also point 3) -MOLA assay): 25 pmoles of phosphorothioate and charge-tag modified primer extension primers (with the sequences 5'-(N)_{Y}TTACTGGACTGAGGTTGCC (SEQ ID N° 33 à 54) and 5'-(N)_{Y}TTACTGGACTGAGGTTGCA (SEQ ID N° 55 à 76) are added with 2 mM MgCl₂, 0,2 mM MnCl₂, 100 µM α-S-ddNTPs (in this case only α-S-ddCTP) and 2 U of Thermosequenase to the PCR product already containing buffer and filled up with water to a 20 µl volume. Also the use of primers that already contain a charge-neutral backbone such as methylphosphonates in combination with dNTPs and/or ddNTPs is possible. An initial denaturing step 2 min at 95°C is used followed by 40 cycles of 20 s at 95°C, 1 min at 58°C and 1 min at 62°C.

In the sequences of the primers, N means the whole variety of bases for example a G-tail, y means a variable number of bases, and can have any value between 0 and 21.

## Claims

1. A method for genotyping of single nucleotide polymorphisms (SNPs) by mass spectrometry, comprising the steps of :
a. reduction of the complexity of the genomic DNA sample to obtain a large representation of the genome by Alu PCR or degenerate oligonucleotide primer PCR (DOP-PCR),
b. generation of (an) allele specific product(s) on the products generated in step a, wherein the generation of allele specific product(s) in step b. is achieved by at least one method that uses (an) allele specific oligonucteotide(s),
c. mass spectrometric analysis of the products generated in step b, wherein the mass spectrometric analysis in step c. is performed on the products generated in step b. without purification or separation from the reaction mixture.

2. The method of claim 1, wherein step b. is performed by at least one method chosen in the group consisting of primer extension, allele specific ligation, and cleavage of an overhang by a cleavase, a flap-endonuclease (FEN), a resolvase or an endonuclease.

3. The method of any of claims 1 to 2, wherein the allele specific oligonucleotide (s) is (are) modified as to allow a mass spectrometric analysis with significantly higher sensitivity than for (an) unmodified oligonucleotide (s).

4. The method of any of claims 1 to 3, wherein the products generated in step b. are or can be conditioned to carry a single excess charge, either positive or negative.

5. The method of any of any of claims 1 to 4, wherein the products generated in step b. and after conditioning are between 2 and 10 bases long.

6. The method of any of claims 1 to 5, wherein at least one oligonucleotide used in step b. is chimeric.

7. The method of claim 6, wherein the chimeric oligonucleotide (s) consists in a regular sugar-phosphate backbone with an end being a phosphorothioate, phosphoroselenoate, methylphosphonate, ethylphosphonate, methoxyphosphonate, ethoxyphosphonate, peptide nucleic acid or a peptide.

8. The method of claim 7, wherein the regular part of the oligonucleotide (s) is between 13 and 40 bases long.

9. The method of any of claims 6 to 8, wherein the products generated in step b. are obtained by cleavage of the modified part from the unmodified part of the chimeric oligonucleotide (s).

10. The method of claim 9, wherein the cleavage is performed by at least one method of the group consisting of an inhibited endonuclease digest, an inhibited exonuclease digest or chemical cleavage.

11. The method of any of claims 1 to 10, wherein step c. is performed by mass analysis of the products of step b., the mass of each generated product being unique and allowing unambiguous allele assignment.

12. The method of any of claims I to 11, wherein step c. is performed by matrix assisted laser desorption/ionization time-of-flight mass spectrometry.

13. The method of claim 12, wherein the matrix is chosen from the group consisting of α-cyano-4-hydroxycinnammic acid, α-cyano-4-hydroxycinnamic acid methyl ester,a-cyano-4-methoxycinnamic acid matrices, derivatives thereof, and a mixture of these matrices.

14. The method of any of claims 1 to 11, wherein step c. is performed by electrospray ionization mass spectrometry.

## Patentansprüche

1. Verfahren zur Genotypisierung von Einzelnukleotid-Polymorphismen (SNPs) durch Massenspektrometrie, umfassend die Schritte:
a. Verringerung der Komplexität der genomischen DNA-Probe, um eine breite Repräsentation des Genoms zu erhalten, mittels Alu-PCR oder degenerierter-Oligonukleotid-Primer-PCR (DOP-PCR),
b. Erzeugung eines Allel-spezifischen Produkts über bei den in Schritt a. erzeugten Allel-spezifischen Produkten, wobei die Erzeugung des bzw. der Allel-spezifischen Produkts bzw. Produkte in Schritt b. durch mindestens ein Verfahren erzielt wird, das (ein) Allel-spezifische(s) Oligonukleotid(e) verwendet,
c. massenspektrometrische Analyse der in Schritt b. erzeugten Produkte, wobei die massenspektrometrische Analyse inschritt c. an den in inschritt b. erzeugten Produkten ohne Reinigung oder Abtrennung aus der Reaktionsmischung durchgeführt wird.

2. Verfahren nach Anspruch 1, bei dem Schritt b. anhand mindestens eines Verfahrens durchgeführt wird, das ausgewählt ist aus der Gruppe : Primer-Verlängerung, Allel-spezifischer Ligierung und Spaltung eines Überhangs durch eine Cleavase, eine Flap-Endonuklease (FEN), eine Resolvase oder eine Endonuklease.

3. Verfahren nach irgendeinem der Ansprüche 1 bis 2, bei dem das bzw. die Allel-spezifische(n) Oligonukleotid(e) so modifiziert wird (werden), dass eine massenspektrometrische Analyse mit signifikant höherer Empfindlichkeit als bei (einem) unmodifizierten Oligonukleotid(en) ermöglicht wird.

4. Verfahren nach irgendeinem der Ansprüche 1 bis 3, bei dem die in Schritt b. erzeugten Produkte so konditioniert werden oder werden können, dass sie eine einzige überschüssige Ladung, entweder positiv oder negativ, tragen.

5. Verfahren nach irgendeinem der Ansprüche 1 bis 4, bei dem die in Schritt b. erzeugten Produkte und nach der Konditionierung zwischen zwei und zehn Basen lang sind.

6. Verfahren nach irgendeinem der Ansprüche 1 bis 5, bei dem mindestens ein in Schritt b. verwendetes Oligonukleotid chimär ist.

7. Verfahren nach Anspruch 6, bei dem das bzw. die chimäre(n) Oligonukleotid(e) aus einer regulären Zucker-Phosphat-Hauptkette besteht, wobei ein Ende ein Phosphorothioat, Phosphoroselenoat, Methylphosphonat, Ethylphosphonat, Methoxyphosphonat, Ethoxyphosphonat, eine Peptid-Nukleinsäure oder ein Peptid ist.

8. Verfahren nach Anspruch 7, bei dem der reguläre Teil des bzw. der Oligonukleotids bzw. Oligonukleotide zwischen 13 und 40 Basen lang ist.

9. Verfahren nach irgendeinem der Ansprüche 6 bis 8, bei dem die in Schritt b. erzeugten Produkte durch die Spaltung des modifizierten Teils von dem unmodifizierten Teil des bzw. der chimären Oligonukleotids bzw. Oligonukleotide erhalten werden.

10. Verfahren nach Anspruch 9, bei dem die Spaltung durch mindestens ein Verfahren aus der Gruppe durchgeführt wird, die aus einem gehemmten Endonuklease-Verdau, einem gehemmten Exonuklease-Verdau oder einer chemischen Spaltung besteht.

11. Verfahren nach irgendeinem der Ansprüche 1 bis 10, bei dem Schritt c. durch Massenanalyse der Produkte von Schritt b. durchgeführt wird, wobei die Masse jedes erzeugten Produkts einzigartig ist und eine zweifelsfreie Allel-Zuordnung ermöglicht.

12. Verfahren nach irgendeinem der Ansprüche 1 bis 11, bei dem Schritt c. durch Matrix-unterstützte Laser-Desorption/lonisation-Time-of-Flight-Massenspektrometrie durchgeführt wird.

13. Verfahren nach Anspruch 12, bei dem die Matrix ausgewählt ist aus der Gruppe bestehend aus a-Cyano-4-hydroxyzimtsäure, a-Cyano-4-hydroxyzimtsäuremethylester, a-Cyano-4-methoxyzimtsäure-Matrizes, deren Derivaten und einer Mischung dieser Matrizes.

14. Verfahren nach irgendeinem der Ansprüche 1 bis 11, bei dem Schritt c. durch Elektrosprayionisations-Massenspektrometrie durchgeführt wird.

## Revendications

1. Procédé pour le génotypage des polymorphismes de nucléotides simples (SNP) par spectrométrie de masse, comprenant les étapes suivantes .
a. réduction de la complexité de l'échantillon d'ADN génomique pour obtenir une large représentation du génome par PCR Alu ou par PCR amorce à amorces oligonucléotidiques dégénérées (DOP-PCR),
b. génération d'un ou de plusieurs produits allèles-spécifiques sur les produits générés à l'étape a, la génération du ou des produits allèles-spécifiques dans l'étape b étant mise en oeuvre par au moins un procédé qui utilise un ou des oligonucléotides allèles-spécifiques,
c. analyse par spectrométrie de masse des produits générés à l'étape b, l'analyse par spectrométrie de l'étape c étant mise en oeuvre sur les produits générés à l'étape b, sans purification, ni séparation à partir du milieu réactionnel.

2. Procédé selon la revendication 1, dans lequel l'étape b est mise en oeuvre par au moins un procédé choisi dans le groupe constitué par ²extension par amorce, ligature allèle-spécifique, et clivage d'une extrémité protubérance par une clivase, une endonucléase « flap » (FEN), une résolvase ou une endonucléase.

3. Procédé selon l'une quelconque des revendications 1 et 2, dans lequel le ou les oligonucléotides allèles-spécifiques (est) sont modifié(s) pour permettre une analyse par spectrométrie de masse avec une sensibilité significativement plus élevée que pour un ou des oligonucléotide(s) non (modifiés).

4. Procédé selon l'une quelconque des revendications 1 à 3, dans lequel les produits générés à l'étape b sont, ou peuvent être, conditionnés pour porter une charge excédentaire unique, qui peut être soit positive, soit négative.

5. Procédé selon l'une quelconque des revendications 1 à 4, dans lequel les produits générés à l'étape b et après conditionnement comprennent entre 2 et 10 bases.

6. Procédé selon l'une quelconque des revendications 1 à 5, dans lequel au moins un oligonucléotide utilisé à l'étape b est chimérique.

7. Procédé selon la revendication 6, dans lequel le ou les oligonucléotides chimériques sont constitués d'un squelette sucre-phosphate régulier avec une extrémité qui est un phosphorothioate, un phosphorosélénoate, un méthylphosphonate, un éthyl-phosphonate, un méthoxyphosphonate, un éthoxyphosphonate, un acide nucléique peptidique ou un peptide.

8. Procédé selon la revendication 7, dans lequel la partie régulière du ou des oligonucléotides chimériques comprend entre 13 et 40 bases.

9. Procédé selon l'une quelconque des revendications 6 à 8, dans lequel les produits générés à l'étape b sont obtenus par clivage de la partie modifiée et de la partie non modifiée du ou des oligonucléotides chimériques.

10. Procédé selon la revendication 9, dans lequel le clivage est obtenu par au moins un procédé choisi dans le groupe constitué par une digestion avec une endonucléase inhibée, une digestion avec une exonucléase inhibée ou un clivage chimique.

11. Procédé selon l'une quelconque des revendications 1 à 10, dans lequel l'étape c est réalisée par analyse de la masse des produits de l'étape b, la masse de chaque produit généré étant unique et permettant l'identification non ambiguë de l'allèle.

12. Procédé selon l'une quelconque des revendications 1 à 11, dans lequel l'étape c est mise en oeuvre par désorption/ionisation laser avec matrice couplée à la spectrométrie de masse à temps de vol.

13. Procédé selon la revendication 12, dans lequel la matrice est choisie dans le groupe constitué par les matrices d'acide α-cyano-4-hydroxycinnamique, d'ester méthylique d'acide α-cyano-4-hydroxycinnamique, d'acide α-cyano-4-méthoxycinnamique, leurs dérivés, et un mélange desdites matrices.

14. Procédé selon l'une quelconque des revendications 1 à 11, dans lequel l'étape c est mise en oeuvre par spectrométrie de masse par ionisation par electronébullisation.


### SEQUENCE LISTING

<110> Centre National de Génotypage
<120> Sample generation for genotyping by mass spectrometry
<130> D18716
<150> EP 00 401 886
   <151> 2000-06-30
<160> 76
<170> PatentIn Ver. 2.1
<210> 1
   <211> 44
   <212> DNA
   <213> Homo sapiens
<400> 1
   cttactggac tgaggttgca cctgctccag ggagcccatg tgac 44
<210> 2
   <211> 44
   <212> DNA
   <213> Homo sapiens
<400> 2
   cttactggac tgaggttgcc cctgctccag ggagcccatg tgac 44
<210> 3
   <211> 38
   <212> DNA
   <213> Homo sapiens
<400> 3
   aatgacctga ctccaacggg gacgaggtcc ctcgggta 38
<210> 4
   <211> 38
   <212> DNA
   <213> Homo sapiens
<400> 4
   aatgacctga ctccaacgtg gacgaggtcc ctcgggta 38
<210> 5
   <211> 38
   <212> DNA
   <213> Homo sapiens
<400> 5
   tactggactg aggttgcccc tgctccaggg agcccatg 38
<210> 6
   <211> 38
   <212> DNA
   <213> Homo sapiens
<400> 6
   tactggactg aggttgcacc tgctccaggg agcccatg 38
<210> 7
   <211> 26
   <212> DNA
   <213> Homo sapiens
<400> 7
   gcatgagaat cgcttgagcc cagccg 26
<210> 8
   <211> 28
   <212> DNA
   <213> Homo sapiens
<400> 8
   ttaagggcaa tacagcaaga ccccgtct 28
<210> 9
   <211> 28
   <212> DNA
   <213> Homo sapiens
<400> 9
   ttatgggcaa tacagcaaga ccccgtct 28
<210> 10
   <211> 28
   <212> DNA
   <213> Homo sapiens
<400> 10
   ttaagggcaa tacagcaaga ccccgtct 28
<210> 11
   <211> 28
   <212> DNA
   <213> Homo sapiens
<400> 11
   ttatgggcaa tacagcaaga ccccgtct 28
<210> 12
   <211> 41
   <212> DNA
   <213> Homo sapiens
<400> 12
   ggccctgtaa ctcggaaggg caatacagca agaccccgtc t 41
<210> 13
   <211> 41
   <212> DNA
   <213> Homo sapiens
<400> 13
   ggaactgtgg ctcttatggg caatacagca agaccccgtc t 41
<210> 14
   <211> 17
   <212> DNA
   <213> Homo sapiens
<400> 14
   ggccctgtaa ctcggag 17
<210> 15
   <211> 17
   <212> DNA
   <213> Homo sapiens
<400> 15
   ggaactgtgg ctcttaa 17
<210> 16
   <211> 46
   <212> DNA
   <213> Homo sapiens
<400> 16
   ggactgaact gccctgtacg acggtccatc cgagaaacag cccctt 46
<210> 17
   <211> 46
   <212> DNA
   <213> Homo sapiens
<400> 17
   ggactgaact gcaatgtacg acactgctta agagccacag ttcctt 46
<210> 18
   <211> 20
   <212> DNA
   <213> Homo sapiens
<400> 18
   ctgtggaccg tcgtacaccg 20
<210> 19
   <211> 21
   <212> DNA
   <213> Homo sapiens
<400> 19
   gctgagcagt gtcgtacatt g 21
<210> 20
   <211> 20
   <212> DNA
   <213> Homo sapiens
<400> 20
   ctgtggaccg tcgtacaccg 20
<210> 21
   <211> 21
   <212> DNA
   <213> Homo sapiens
<400> 21
   gctgagcagt gtcgtacatt g 21
<210> 22
   <211> 70
   <212> DNA
   <213> Homo sapiens
<220>
   <223> n means a, g, c ou t/u
<400> 22
   gggcaataca gcaagnnnnn nnnnnnnnnn nnnnnnnnnn nnnnnnnnnn nnnnngcttg 60
   agcccagcca 70
<210> 23
   <211> 20
   <212> DNA
   <213> Homo sapiens
<400> 23
   acgagccggt ctgaatagta 20
<210> 24
<211> 23
   <212> DNA
   <213> Homo sapiens
<220>
   <223> n means a, g, c ou t/u
<400> 24
   ttctcgccgg ccgcnnnnnn atg 23
<210> 25
   <211> 31
   <212> DNA
   <213> Homo sapiens
<400> 25 aagtcgcggg ccgcttgcag tgagccgaga t 31
<210> 26
   <211> 11
   <212> DNA
   <213> Homo sapiens
<400> 26
   gatccagtcc g 11
<210> 27
   <211> 17
   <212> DNA
   <213> Homo sapiens
<400> 27
   ctcgtagact gcgtacc 17
<210> 28
   <211> 18
   <212> DNA
   <213> Homo sapiens
<400> 28
   aattggtacg cagtctac 18
<210> 29
   <211> 16
   <212> DNA
   <213> Homo sapiens
<400> 29
   gacgatgagt cctgag 16
<210> 30
   <211> 14
   <212> DNA
   <213> Homo sapiens
<900> 30
   tactcaggac tcat 14 <210> 31

   <211> 20
   <212> DNA
   <213> Homo sapiens
<220>
   <223> n means a, g, c ou t/u
<400> 31
   gacctgcgta ccaattcnnn 20
<210> 32
   <211> 19
   <212> DNA
   <213> Homo sapiens
<220>
   <223> n means a, g, c ou t/u
<400> 32
   gatgagtcct gagtaannn 19
<210> 33
   <211> 19
   <212> DNA
   <213> Artificial Sequence
<220>
   <223> Description of Artificial Sequence:Primer for SNP of human GM-CSF.
<400> 33
   ttactggact gaggttgcc 19
<210> 34
   <211> 20
   <212> DNA
   <213> Artificial Sequence
<220>
   <223> Description of Artificial Sequence:Primer for SNP of human GM-CSF.
<220>
   <223> n means a, g, c ou t/u
<400> 34
   nttactggac tgaggttgcc 20
<210> 35
   <211> 21
   <212> DNA
   <213> Artificial Sequence
<220>
   <223> Description of Artificial Sequence:Primer for SNP of human GM-CSF.
<220>
   <223> n means a, g, c ou t/u
<400> 35
   nnttactgga ctgaggttgc c 21
<210> 36
   <211> 22
   <212> DNA
   <213> Artificial Sequence
<220>
   <223> Description of Artificial Sequence:Primer for SNP of human GM-CSF.
<220>
   <223> n means a, g, c ou t/u
<400> 36
   nnnttactgg actgaggttg cc 22
<210> 37
   <211> 23
   <212> DNA
   <213> Artificial Sequence
<220>
   <223> Description of Artificial Sequence:Primer for SNP of human GM-CSF.
<220>
   <223> n means a, g, c ou t/u
<400> 37
   nnnnttactg gactgaggtt gcc 23
<210> 38
   <211> 24
   <212> DNA
   <213> Artificial Sequence
<220>
   <223> Description of Artificial Sequence:Primer for SNP of human GM-CSF.
<220>
   <223> n means a, g, c ou t/u
<400> 38
   nnnnnttact ggactgaggt tgcc 24
<210> 39
   <211> 25
   <212> DNA
   <213> Artificial Sequence
<220>
   <223> Description of Artificial Sequence:Primer for SNP of human GM-CSF.
<220>
   <223> n means a, g, c ou t/u
<400> 39
   nnnnnnttac tggactgagg ttgcc 25
<210> 40
   <211> 26
   <212> DNA
   <213> Artificial Sequence
<220>
   <223> Description of Artificial Sequence:Primer for SNP of human GM-CSF.
<220>
   <223> n means a, g, c ou t/u
<400> 40
   nnnnnnntta ctggactgag gttgcc 26
<210> 41
   <211> 27
   <212> DNA
   <213> Artificial Sequence
<220>
   <223> Description of Artificial Sequence:Primer for SNP of human GM-CSF.
<220>
   <223> n means a, g, c ou t/u
<400> 41
   nnnnnnnntt actggactga ggttgcc 27
<210> 42
   <211> 28
   <212> DNA
   <213> Artificial Sequence
<220>
   <223> Description of Artificial Sequence:Primer for SNP of human GM-CSF.
<220>
   <223> n means a, g, c ou t/u
<400> 42
   nnnnnnnnnt tactggactg aggttgcc 28
<210> 43
   <211> 29
   <212> DNA
   <213> Artificial Sequence
<220>
   <223> Description of Artificial Sequence:Primer for SNP of human GM-CSF.
<220>
   <223> n means a, g, c ou t/u
<400> 43
   nnnnnnnnnn ttactggact gaggttgcc 29
<210> 44
   <211> 30
   <212> DNA
   <213> Artificial Sequence
<220>
   <223> Description of Artificial Sequence:Primer for SNP of human GM-CSF.
<220>
   <223> n means a, g, c ou t/u
<400> 44
   nnnnnnnnnn nttactggac tgaggttgcc 30
<210> 45
   <211> 31
   <212> DNA
   <213> Artificial Sequence
<220>
   <223> Description of Artificial Sequence:Primer for SNP of human GM-CSF.
<220>
   <223> n means a, g, c ou t/u
<400> 45
   nnnnnnnnnn nnttactgga ctgaggttgc c 31
<210> 46
   <211> 32
   <212> DNA
   <213> Artificial Sequence
<220>
   <223> Description of Artificial Sequence:Primer for SNP of human GM-CSF.
<220>
   <223> n means a, g, c ou t/u
<400> 46
   nnnnnnnnnn nnnttactgg actgaggttg cc 32
<210> 47
   <211> 33
   <212> DNA
   <213> Artificial Sequence
<220>
   <223> Description of Artificial Sequence:Primer for SNP of human GM-CSF.
<220>
   <223> n means a, g, c ou t/u
<400> 47
   nnnnnnnnnn nnnnttactg gactgaggtt gcc 33
<210> 48
   <211> 34
   <212> DNA
   <213> Artificial Sequence
<220>
   <223> Description of Artificial Sequence:Primer for SNP of human GM-CSF.
<220>
   <223> n means a, g, c ou t/u
<400> 48
   nnnnnnnnnn nnnnnttact ggactgaggt tgcc 34
<210> 49
   <211> 35
   <212> DNA
   <213> Artificial Sequence
<220>
   <223> Description of Artificial Sequence:Primer for SNP of human GM-CSF.
<220>
   <223> n means a, g, c ou t/u
<400> 49
   nnnnnnnnnn nnnnnnttac tggactgagg ttgcc 35
<210> 50
   <211> 36
   <212> DNA
   <213> Artificial Sequence
<220>
   <223> Description of Artificial Sequence:Primer for SNP of human GM-CSF.
<220>
   <223> n means a, g, c ou t/u
<400> 50
   nnnnnnnnnn nnnnnnntta ctggactgag gttgcc 36
<210> 51
   <211> 37
   <212> DNA
   <213> Artificial Sequence
<220>
   <223> Description of Artificial Sequence:Primer for SNP of human GM-CSF.
<220>
   <223> n means a, g, c ou t/u
<400> 51
   nnnnnnnnnn nnnnnnnntt actggactga ggttgcc 37
<210> 52
   <211> 38
   <212> DNA
   <213> Artificial Sequence
<220>
   <223> Description of Artificial Sequence:Primer for SNP of human GM-CSF.
<220>
   <223> n means a, g, c ou t/u
<400> 52
   nnnnnnnnnn nnnnnnnnnt tactggactg aggttgcc 38
<210> 53
   <211> 39
   <212> DNA
   <213> Artificial Sequence
<220>
   <223> Description of Artificial Sequence:Primer for SNP of human GM-CSF.
<220>
   <223> n means a, g, c ou t/u
<400> 53
   nnnnnnnnnn nnnnnnnnnn ttactggact gaggttgcc 39
<210> 54
   <211> 40
   <212> DNA
   <213> Artificial Sequence
<220>
   <223> Description of Artificial Sequence:Primer for SNP of human GM-CSF.
<220>
   <223> n means a, g, c ou t/u
<400> 54
   nnnnnnnnnn nnnnnnnnnn nttactggac tgaggttgcc 40
<210> 55
   <211> 19
   <212> DNA
   <213> Artificial Sequence
<220>
   <223> Description of Artificial Sequence:Primer for SNP of human GM-CSF.
<400> 55
   ttactggact gaggttgca 19
<210> 56
   <211> 20
   <212> DNA
   <213> Artificial Sequence
<220>
   <223> Description of Artificial Sequence:Primer for SNP of human GM-CSF.
<220>
   <223> n means a, g, c ou t/u
<400> 56
   nttactggac tgaggttgca 20
<210> 57
   <211> 21
   <212> DNA
   <213> Artificial Sequence
<220>
   <223> Description of Artificial Sequence:Primer for SNP of human GM-CSF.
<220>
   <223> n means a, g, c ou t/u
<400> 57
   nnttactgga ctgaggttgc a 21
<210> 58
   <211> 22
   <212> DNA
   <213> Artificial Sequence
<220>
   <223> Description of Artificial Sequence:Primer for SNP of human GM-CSF.
<220>
   <223> n means a, g, c ou t/u
<400> 58
   nnnttactgg actgaggttg ca 22
<210> 59
   <211> 23
   <212> DNA
   <213> Artificial Sequence
<220>
   <223> Description of Artificial Sequence:Primer for SNP of human GM-CSF.
<220>
   <223> n means a, g, c ou t/u
<400> 59
   nnnnttactg gactgaggtt gca 23
<210> 60
   <211> 24
   <212> DNA

   <213> Artificial Sequence
<220>
   <223> Description of Artificial Sequence:Primer for SNP of human GM-CSF.
<220>
   <223> n means a, g, c ou t/u
<400> 60
   nnnnnttact ggactgaggt tgca 24
<210> 61
   <211> 25
   <212> DNA
   <213> Artificial Sequence
<220>
   <223> Description of Artificial Sequence:Primer for SNP of human GM-CSF.
<220>
   <223> n means a, g, c ou t/u
<400> 61
   nnnnnnttac tggactgagg ttgca 25
<210> 62
   <211> 26
   <212> DNA
   <213> Artificial Sequence
<220>
   <223> Description of Artificial Sequence:Primer for SNP of human GM-CSF.
<220>
   <223> n means a, g, c ou t/u
<400> 62
   nnnnnnntta ctggactgag gttgca 26
<210> 63
   <211> 27
   <212> DNA
   <213> Artificial Sequence
<220>
   <223> Description of Artificial Sequence:Primer for SNP of human GM-CSF.
<220>
   <223> n means a, g, c ou t/u
<400> 63
   nnnnnnnntt actggactga ggttgca 27
<210> 64
   <211> 28
   <212> DNA
   <213> Artificial Sequence
<220>
   <223> Description of Artificial Sequence:Primer for SNP of human GM-CSF.
<220>
   <223> n means a, g, c ou t/u
<400> 64
   nnnnnnnnnt tactggactg aggttgca 28
<210> 65
   <211> 29
   <212> DNA
   <213> Artificial Sequence
<220>
   <223> Description of Artificial Sequence:Primer for SNP of human GM-CSF.
<220>
   <223> n means a, g, c ou t/u
<400> 65
   nnnnnnnnnn ttactggact gaggttgca 29
<210> 66

   <211> 30
   <212> DNA
   <213> Artificial Sequence
<220>
   <223> Description of Artificial Sequence:Primer for SNP of human GM-CSF.
<220>
   <223> n means a, g, c ou t/u
<400> 66
   nnnnnnnnnn nttactggac tgaggttgca 30
<210> 67

   <211> 31
   <212> DNA
   <213> Artificial Sequence
<220>
   <223> Description of Artificial Sequence:Primer for SNP of human GM-CSF.
<220>
   <223> n means a, g, c ou t/u
<400> 67
   nnnnnnnnnn nnttactgga ctgaggttgc a 31
<210> 68
   <211> 32
   <212> DNA
   <213> Artificial Sequence
<220>
   <223> Description of Artificial Sequence:Primer for SNP of human GM-CSF.
<220>
   <223> n means a, g, c ou t/u
<400> 68
   nnnnnnnnnn nnnttactgg actgaggttg ca 32
<210> 69
   <211> 33
   <212> DNA
   <213> Artificial Sequence
<220>
   <223> Description of Artificial Sequence:Primer for SNP of human GM-CSF.
<220>
   <223> n means a, g, c ou t/u
<400> 69
   nnnnnnnnnn nnnnttactg gactgaggtt gca 33 <210> 70

   <211> 34
   <212> DNA
   <213> Artificial Sequence
<220>
   <223> Description of Artificial Sequence:Primer for SNP of human GM-CSF.
<220>
   <223> n means a, g, c ou t/u
<400> 70 nnnnnnnnnn nnnnnttact ggactgaggt tgca 34
<210> 71
   <211> 35
   <212> DNA
   <213> Artificial Sequence
<220>
   <223> Description of Artificial Sequence:Primer for SNP of human GM-CSF.
<220>
   <223> n means a, g, c ou t/u
<400> 71
   nnnnnnnnnn nnnnnnttac tggactgagg ttgca 35
<210> 72
   <211> 36
   <212> DNA
   <213> Artificial Sequence
<220>
   <223> Description of Artificial Sequence:Primer for SNP of human GM-CSF.
<220>
   <223> n means a, g, c ou t/u
<400> 72
   nnnnnnnnnn nnnnnnntta ctggactgag gttgca 36
<210> 73
   <211> 37
   <212> DNA
   <213> Artificial Sequence
<220>
   <223> Description of Artificial Sequence:Primer for SNP of human GM-CSF.
<220>
   <223> n means a, g, c ou t/u
<400> 73
   nnnnnnnnnn nnnnnnnntt actggactga ggttgca 37
<210> 74
   <211> 38
   <212> DNA
   <213> Artificial Sequence
<220>
   <223> Description of Artificial Sequence:Primer for SNP of human GM-CSF.
<220>
   <223> n means a, g, c ou t/u
<400> 74
   nnnnnnnnnn nnnnnnnnnt tactggactg aggttgca 38
<210> 75
   <211> 39
   <212> DNA
   <213> Artificial Sequence
<220>
   <223> Description of Artificial Sequence:Primer for SNP of human GM-CSF.
<220>
   <223> n means a, g, c ou t/u
<400> 75
   nnnnnnnnnn nnnnnnnnnn ttactggact gaggttgca 39
<210> 76
   <211> 40
   <212> DNA
   <213> Artificial Sequence
<220>
   <223> Description of Artificial Sequence:Primer for SNP of human GM-CSF.
<220>
   <223> n means a, g, c ou t/u
<400> 76
   nnnnnnnnnn nnnnnnnnnn nttactggac tgaggttgca 40